# EUROPEAN PATENT SPECIFICATION

(11) **EP 1 920 703 B1**
(45) Date of publication and mention of the grant of the patent: **23.03.2011**
(21) Application number: 06796983.2
(22) Date of filing: 29.08.2006
(51) Int. Cl.: A61B 1/00, A61B 5/07

(54) **IN-EXAMINER INFORMATION ACQUISITION SYSTEM**
INFORMATIONSERFASSUNGSSYSTEM IN EINEM UNTERSUCHTEN
SYSTEME D ACQUISITION D INFORMATIONS AUPRES D UNE PERSONNE EXAMINEE

(30) Priority: 29.08.2005 JP 2005248012
(43) Date of publication of application: 14.05.2008
(60) Divisional application: 08011262.6
(73) Proprietor: OLYMPUS MEDICAL SYSTEMS CORP., Shibuya-ku Tokyo 151-0072 (JP); Olympus Corporation, Tokyo 151-0072 (JP)
(72) Inventor: SHIGEMORI, Toshiaki c/o OLYMPUS MEDICAL SYSTEMS CORP.,, Tokyo 151-0072 (JP); FUJITA, Manabu c/o OLYMPUS MEDICAL SYSTEMS CORP.,, Tokyo 151-0072 (JP); NAGASE, Ayako c/o OLYMPUS MEDICAL SYSTEMS CORP.,, Tokyo 151-0072 (JP); MATSUI, Akira c/o OLYMPUS CORPORATION,, Tokyo 151-0072 (JP); NAKATSUCHI, Kazutaka c/o OLYMPUS MEDICAL SYSTEMS CORP.,, Tokyo 151-0072 (JP)
(74) Representative: von Hellfeld, Axel
(86) International application number: PCT/JP2006/317006
(87) International publication number: WO 2007/026712

(56) References cited:
- JP-A- 2004 267 350
- JP-A- 2004 328 941
- US-A- 4 556 061
- US-A1- 2004 215 084
- US-A1- 2005 007 073

## Description

### TECHNICAL FIELD

The present invention relates to an intra-subject information acquiring system that notifies a remaining power amount of a body-insertable apparatus, such as a capsule endoscope, which is inserted into a subject and acquires intra-subject information.

### BACKGROUND ART

In recent years, in the field of endoscope, a capsule endoscope having an imaging function and a radio communication function has appeared. After a subject swallows the capsule endoscope from the mouth to perform an observation (examination), the capsule endoscope moves within internal organs (within the body cavity) such as an esophagus, a stomach, and a small intestine, following its peristalsis, during the observation period, until the capsule endoscope is naturally discharged from the body (human body) of the subject. The capsule endoscope sequentially images at a predetermined imaging rate, using the imaging function.

During the observation period when the capsule endoscope moves within the internal organs, image data acquired by imaging within the body cavity by the capsule endoscope is sequentially transmitted to the outside of the subject, by the radio communication function such as radio transmission, and is stored into a memory provided within an external receiving apparatus. When the subject carries the receiving apparatus having the radio communication function and the memory function, the subject can move freely even during the observation period after the subject swallows the capsule endoscope until the capsule endoscope is discharged. After the observation, a doctor or a nurse can perform diagnosis by displaying the images of the body cavity on a displaying unit such as a display, based on image data stored in the memory of the receiving device (for example, see Patent Document 1).
With regard to the supply of power, in view of the fact that the capsule endoscope works inside a living body, some battery supply systems employ a battery which is mounted in the capsule endoscope and supplies the power to the capsule endoscope, while other power transmission systems transmit power from outside the living body to the capsule endoscope.
The latter power transmission system includes a mechanism which is provided with a power receiving antenna inside and transmits the power to the capsule endoscope that remains within the living body through the power receiving antenna (see Patent Document 1).
Patent Document 1: Japanese Patent Application Laid-open No. 2001-231186
Document US 4,556,061 concerns a cardiac pacer comprising a battery consumption monitor circuit. The pacer comprises a battery and an indication of consumed battery capacity determined by battery consumption monitor circuit is provided via an antenna to an external readout.
Document 2004/0215084 A1 concerns a wireless in-vivo information acquiring system and an external device. The system comprises a system control circuit controlling the drive of the units within the system by using power stored in a capacitor and a power supply state for causing the units to drive. A level determining signal and in-vivo information are intermittently transmitted to an external device.

### DISCLOSURE OF INVENTION

### PROBLEM TO BE SOLVED BY THE INVENTION

Since some of the above power transmission systems are configured merely to supply power from the outside of the living body, however, an amount of power remaining in a capsule endoscope cannot be known.
The present invention has been achieved in view of the above, and it is an object of the invention to provide an intra-subject information acquiring system that allows a user to promptly recognize a remaining power amount of a body-insertable apparatus.
Some or all of the above problems are overcome by an intra-subject information acquiring system having the features of claim 1.

### MEANS FOR SOLVING PROBLEM

An intra-subject information acquiring system according to one aspect of the present invention includes: a body-insertable apparatus that includes a function executing unit that executes a predetermined function of acquiring intra-subject information, a power supply unit that supplies power to the function executing unit, a recognizing unit that recognizes a remaining power amount of the power supply unit, a superimposing unit that superimposes remaining-power-amount information recognized by the recognizing unit on a transmission signal, and a transmitting unit that transmits the transmission signal on which remaining-power-amount information is superimposed by the superimposing unit; and an external device that includes a receiving unit that receives the transmission signal transmitted from the transmitting unit, a detector that detects the remaining-power-amount information from the transmission signal received by the receiving unit, and a displaying unit that displays remaining-power-amount information detected by the detector.
In the intra-subject information acquiring system, the body-insertable apparatus further includes a power storage unit that secures the power supply by radio power supply from outside.

### EFFECT OF THE INVENTION

According to the intra-subject information acquiring system of the present invention, a recognizing unit of a body-insertable apparatus recognizes a remaining power amount. The remaining-power-amount information is transmitted to an external device, and is displayed on a displaying unit. Therefore, there is an effect that the remaining power amount of the body-insertable apparatus can be promptly recognized.

### BRIEF DESCRIPTION OF DRAWINGS

FIG. 1 is a schematic diagram of an overall configuration of a radio intra-subject information acquiring system according to the present invention;
FIG. 2 is a block diagram of an internal configuration of a capsule endoscope according to the present invention;
FIG. 3 is a block diagram of a configuration of a remaining-power-amount recognizing circuit shown in FIG. 2 and its periphery according to a first embodiment not forming part of the claimed invention;
FIG. 4 is a block diagram of an internal configuration of a receiving apparatus according to the present invention;
FIG. 5 is a block diagram of a configuration of relevant parts of an external device including an RF receiving unit; and
FIG. 6 is a block diagram of a configuration of a remaining-power-amount recognizing circuit and its periphery according to a second embodiment forming the claimed invention.

### EXPLANATIONS OF LETTERS OR NUMERALS

- 1: Subject
- 2: Capsule endoscope
- 3: Receiving apparatus
- 4: Display device
- 5: Portable recording medium
- 11: LED
- 12: LED driving circuit
- 13: CCD
- 14: CCD driving circuit
- 15: RF transmitting unit
- 16: RF antenna
- 17: Power receiving antenna
- 18: Separating circuit
- 19: Power reproducing circuit
- 20: Remaining-power-amount recognizing circuit
- 20a: A/D converter
- 20b: Digital processing circuit
- 20c: Inverter
- 21: Booster circuit
- 22: Capacitor
- 23: System control circuit
- 24: Control-information detecting circuit
- 31: Transmitting/receiving jacket
- 32: External device
- 39: Power supply unit
- 40: Power-supply control circuit
- 41: Oscillator
- 42: Control-information input unit
- 43: Superimposing circuit
- 44: Amplifier circuit
- 45: Switching circuit
- 46: RF receiving unit
- 46a: RF-BB converting circuit
- 46b: Binarizing circuit
- 46c: Synchronization detecting circuit
- 46d: Serial/parallel converting circuit
- 47: Image processing unit
- 48: Remaining-power-amount detecting circuit
- 49: Storage unit
- 50: Control circuit
- 51: Display unit
- A1 to An: Receiving antenna
- B1 to Bm: Power supply Antenna
- R1, R2: Resistor

### BEST MODE(S) FOR CARRYING OUT THE INVENTION

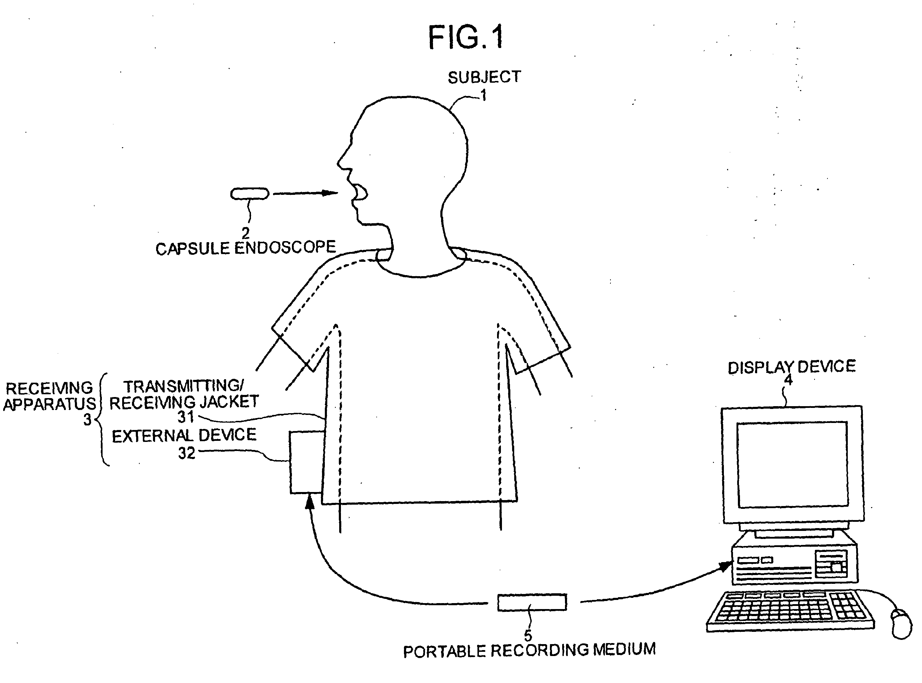

Exemplary embodiments of an intra-subject information acquiring system will be explained below in detail with reference to the drawings of FIG. 1 to FIG. 6.

### First Embodiment

FIG. 1 is a schematic diagram of an overall configuration of a radio intra-subject information acquiring system according to the present invention. In the following description of the radio intra-subject information acquiring system, a capsule endoscope is explained as one example of the body-insertable apparatus. In FIG. 1, the radio intra-subject information acquiring system includes a receiving apparatus 3 that has a radio receiving function, and a capsule endoscope (a body-insertable apparatus) 2 that is inserted into a subject 1, images a body cavity, and transmits data such as an image data to the receiving apparatus 3 outside the subject 1. The radio intra-subject information acquiring system also includes a display device 4 that displays a body-cavity image based on an image signal received by the receiving apparatus 3, and a portable recording medium 5 that delivers data between the receiving apparatus 3 and the display device 4. The receiving apparatus 3 includes a transmitting/receiving jacket 31 which the subject 1 wears, and an external device 32 that processes a received radio signal.

The display device 4 displays a body-cavity image picked up by the capsule endoscope 2, and has a configuration like a workstation that displays an image based on data acquired by the portable recording medium 5. Specifically, the display device 4 can be configured to directly display an image using a CRT display, a liquid crystal display, or the like, or can be configured to output an image to other medium such as a printer.

The portable recording medium 5 is attachable to and detachable from the external device 32 and the display device 4, and can output or record information when the portable recording medium 5 is mounted on one of the external device 32 and the display device 4. In the present embodiment, while the capsule endoscope 2 moves within the body cavity of the subject 1, the portable recording medium 5 is mounted on the external device 32, and records data transmitted from the capsule endoscope 2. After the capsule endoscope 2 is discharged from the subject 1, that is, after the imaging inside the subject 1 ends, the portable recording medium 5 is taken out from the external device 32, and is mounted on the display device 4. The display device 4 reads the data recorded on the portable recording medium 5. When the portable recording medium 5 configured by a Compact Flash (Registered Trademark) memory or the like delivers data between the external device 32 and the display device 4, the subject 1 can move more freely during the imaging inside the body cavity than when the external device 32 is directly connected to the display device 4 by wire. While the portable recording medium 5 is used to deliver data between the external device 32 and the display device 4, the use of the portable recording medium 5 is not necessarily the only method. Other recording apparatuses, such as a hard disk, built in the external device 32 can be also used, and the recording apparatus and the display device 4 can be connected to each other by radio or by wire to deliver data therebetween.

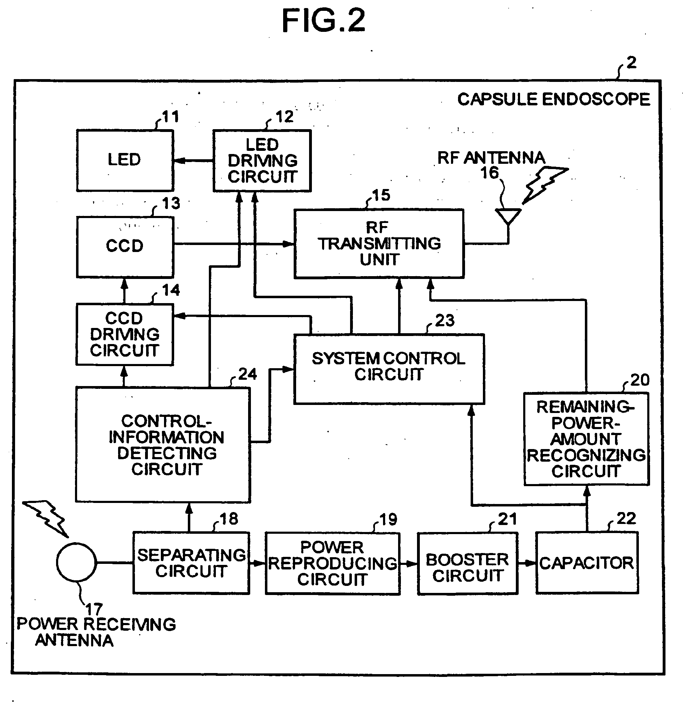

The capsule endoscope 2 is explained next. FIG. 2 is a block diagram of an internal configuration of a capsule endoscope according to the present embodiment. In FIG. 2, the capsule endoscope 2 includes: a light emitting element (LED) 11 (a function executing unit) as an illuminating unit that irradiates an examined part within a body cavity of the subject 1; an LED driving circuit 12 that controls a driving state of the LED 11; a charge-coupled device (CCD) 13 (a function executing unit) as an imaging unit that picks up an image within the body cavity as a reflection light from an area irradiated by the LED 11; a CCD driving circuit 14 that controls a driving state of the CCD 13; an RF transmitting unit 15 (a transmitting unit) that modulates an image signal output from the CCD 13, into an RF signal; and an RF antenna 16 (a transmitting unit) as a transmitting antenna that radio transmits an RF signal output from the RF transmitting unit 15.

The capsule endoscope 2 further includes: a power receiving antenna 17 that receives a radio signal transmitted from the receiving apparatus 3; a separating circuit 18 that separates a power supply signal from a signal received by the power receiving antenna 17; a power reproducing circuit 19 that reproduces power supply (power) from the power supply signal; a booster circuit 21 that boosts the reproduced power; a capacitor 22 (a power storage unit) that stores the boosted power supply; a remaining-power-amount recognizing circuit 20 (a recognizing unit, a superimposing unit) that recognizes a remaining power amount of the capacitor 22; and a system control circuit 23 (a function executing unit, a power supply unit) that controls each unit within the capsule endoscope 2 such as the LED 11 and the CCD 13, using power stored in the capacitor 22. In order to acquire intra-subject information, the LED 11, the CCD 13, the RF transmitting unit 15, and the system control circuit 23 execute predetermined functions of the capsule endoscope 2.

The CCD 13 acquires an intra-subject image as a result of light irradiation by the LED 11 as a light emitting element, and outputs the intra-subject image as an image signal of acquired intra-subject information, to the RF transmitting unit 15. The RF transmitting unit 15 modulates the image signal transmitted from the CCD 13, and radio transmits the modulated image signal as a transmission signal, to the outside of the capsule endoscope 2 via the RF antenna 16 as a transmission antenna. In the present invention, instead of the CCD 13, other imaging elements such as a CMOS sensor can be used. The transmission signal can be living-body information such as temperature information and pH information within the body cavity, instead of the body-cavity image of the subject.

The power receiving antenna 17 is made of a single coil member, and receives a power supply wave transmitted from the receiving apparatus 3 described latter. After the power receiving antenna 17 receives the power supply wave, the separating circuit 18 separates a power supply signal from the power supply wave. The power reproducing circuit 19 reproduces the power supply signal as a power source. The booster circuit 21 boosts the power, and stores the boosted power into the capacitor 22 as power. The system control unit 23 controls the driving of each electric device such as the LED driving circuit 12, the CCD driving circuit 14, and the RF transmission unit 15, using the power stored in the capacitor 22. The system control unit 23 also controls a power supply state to drive each electric device.

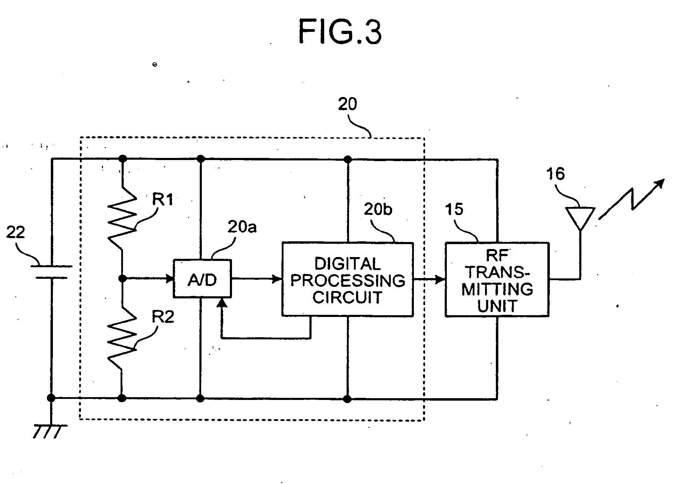

Next, the remaining-power-amount recognizing circuit 20 is explained. FIG. 3 is a block diagram showing a configuration of the remaining-power-amount recognizing circuit 20 not according to the present invention shown in FIG. 2 and its periphery. In FIG. 3, the remaining-power-amount recognizing circuit 20 includes resistors R1 and R2 that resistance-divides the voltage of the capacitor 22, and an A/D converter 20a (an identifying unit) that converts the resistance-divided voltage from an analog signal into a digital signal, and a digital processing circuit 20b (a superimposing unit) that digitally processes a voltage signal from the A/D converter 20a, superimposes the digital signal on the transmission signal, and outputs the superimposed signal to the RF transmitting unit 15.

The digital processing circuit 20b can superimpose only the voltage signal from the A/D converter 20a on a transmission signal, or can superimpose the voltage signal together with white-balance information and a capsule ID (identification signal) on a transmission signal. The transmission signal on which the voltage signal is superimposed can be an image signal, or can be any living-body information radio transmitted from the capsule endoscope 2 to the receiving apparatus 3.

The digital processing circuit 20b can also timer-control the starting of the A/D converter 20a so as to intermittently start the A/D converter 20a to take in a voltage signal, in addition to achieving the function of superimposing the voltage signal on the transmission signal. Based on this configuration, recognition precision of remaining power amount increases, and power consumption of the capacitor 22 can be decreased based on the intermittent control.

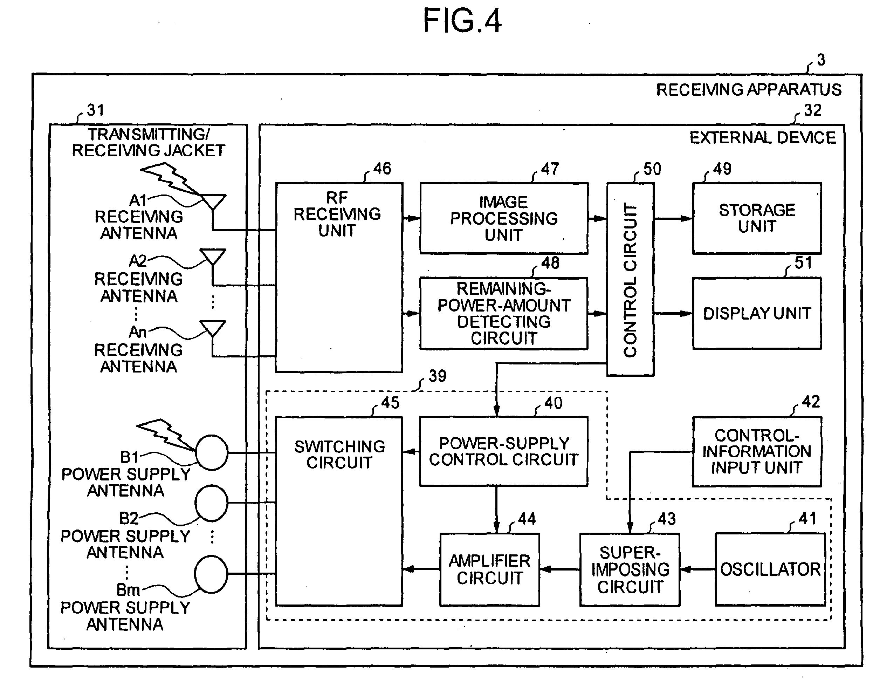

The receiving apparatus 3 is explained next. FIG. 4 is a block diagram of an internal configuration of the receiving apparatus 3 according to the embodiment. In FIG. 4, the receiving apparatus 3 has a shape to enable the subject 1 to wear the receiving apparatus 3, and includes the transmitting/receiving jacket 31 having receiving antennas A1 to An, and the external device 32 that processes a radio signal received via the transmitting/receiving jacket 31. The receiving antennas A1 to An are detachably connected to a connector not shown. The receiving antennas A1 to An can be directly adhered to the external surface of the subject 1, and are not necessarily be provided on the transmitting/receiving jacket 31, and are detachable from the transmitting/receiving jacket 31.

The transmitting/receiving jacket 31 includes the receiving antennas A1 to An, and plural power supply antennas B1 to Bm. The symbols n and m denote natural numbers. Each antenna of the transmitting/receiving jacket 31 is disposed at a predetermined position on the external surface of the subject 1 when the antenna is mounted on the living body as the subject 1. When intra-subject information is to be acquired from one or all of an esophagus, a stomach, a small intestine, and a large intestine, the transmitting/receiving jacket 31 is disposed on the breast or abdomen, or across both the breast and abdomen.

The external device 32 includes an RF receiving unit 46 that executes a predetermined signal processing such as a demodulation of radio signals received by the receiving antennas A1 to An, and that extracts image information acquired by the capsule endoscope 2 from the radio signals, an image processing unit 47 that executes necessary image processing to the extracted image information, a storage unit 49 as a recording unit that records the image-processed image information, and a control circuit 50 that controls each unit. The external device 32 performs signal processing of a radio signal (a transmission signal) transmitted from the capsule endoscope 2.

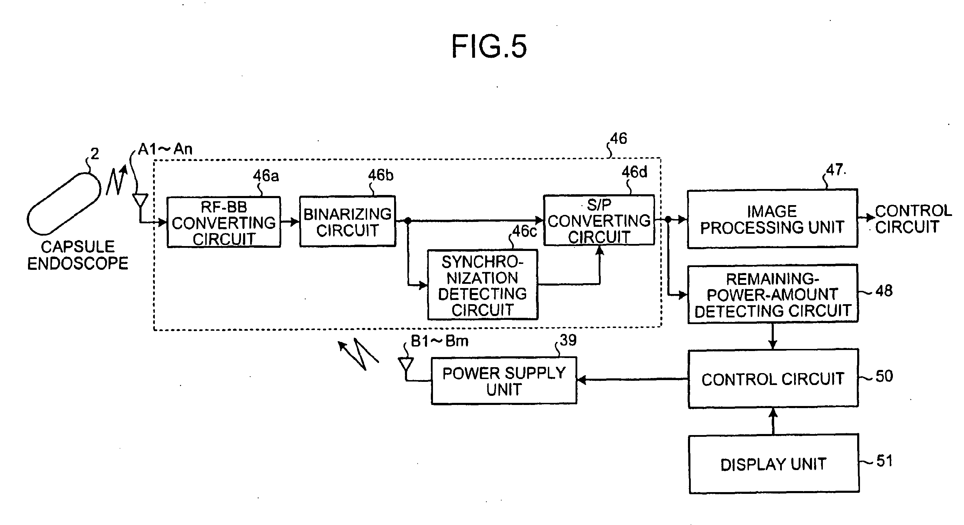

In the embodiment, as shown in FIG. 5, the RF receiving unit 46 includes: an RF-BB converting circuit 46a that converts an RF signal into a baseband signal; a binarizing circuit 46b that amplifies a baseband signal having a weak signal level and filters a frequency band, and generates a signal of a level that can be processed at a latter stage; a synchronization detecting circuit 46c that executes a synchronization detection based on an output signal of the binarizing circuit 46b; and a serial/parallel converting circuit 46d that converts an output signal of the binarizing circuit 46b from a serial signal into a parallel signal. The image information is stored into the portable recording medium 5 via the image processing unit 47 and the storage unit 49.

The external device 32 further includes: a remaining-power-amount detecting circuit 48 (a detector) that detects information of a remaining power amount in the capacitor 22 of the capsule endoscope 2; a power supply unit 39 that supplies power based on the remaining-power-amount information from the control circuit 50; and a display unit 51 (a displaying unit) that displays an input remaining power amount. The remaining-power-amount detecting circuit 48 detects the remaining-power-amount information superimposed on the output signal of the serial/parallel converting circuit 46d, and outputs this information to the control circuit 50. The control circuit 50 changes the remaining-power-amount information detected by the remaining-power-amount detecting circuit 48 into a signal format that can be displayed on the display unit 51, and outputs the remaining-power-amount information to the power supply unit 39.

The display unit 51 has a configuration that directly displays information into a liquid crystal display such as an LCD, for example, and the display unit 51 directly displays a remaining power amount of the capacitor 22 of the capsule endoscope 2 input from the control circuit 50, on a screen of the LCD. The display unit 51 can be configured to output information to other medium such as a printer, as far as the user can recognize the information.

As shown in FIG. 4, the power supply unit 39 includes a superimposing circuit 43 connected to a latter stage of an oscillator 41. In the superimposing circuit 43, control information input from a control-information input unit 42 is superimposed on the power supply signal output from the oscillator 41. An amplifier circuit 44 connected to a latter stage of the superimposing circuit 43 amplifies the power supply signal on which the control information is superimposed, and outputs the amplified signal to a switching circuit 45. A power-supply control circuit 40 receives the remaining-power-amount information input from the control circuit 50, and changes an amplification rate of the power supply signal amplified by the amplifier circuit 44, based on the remaining power amount.

In other words, when the remaining-power-amount information from the control circuit 50 indicates a relatively large amount, for example, the power-supply control circuit 40 controls to suppress the amplification rate of the power supply signal. When the remaining-power-amount information indicates a relatively small amount, the power-supply control circuit 40 controls to increase the amplification rate of the power supply signal. The power supply signal input to the switching circuit 45 is radio transmitted from the power supply antenna of the transmitting/receiving jacket 31.

Assume that the control-information input unit 42 inputs an instruction to change the imaging rate of the CCD 13 from a first imaging rate to a second imaging rate. The superimposing circuit 43 superimposes a frame-number-change information signal transmitted from the control-information input unit 42, on the power supply signal, and inputs the superimposed signal to the amplifier circuit 44. The power-supply control circuit 40 controls to change the amplification rate, and the amplifier circuit 44 amplifies the signal at a predetermined amplification rate. The switching circuit 45 transmits the signal to an optimum power supply antenna among the power supply antennas B1 to Bm, and radio transmits the signal from the power supply antenna. The control-information input unit 42 according to the present embodiment can input various kinds of information. For example, the control-information input unit 42 can input: frame-number change information as information for changing an imaging rate of the CCD 13, that is, a number of frames imaged within a predetermined period of time; LED light-on time information for changing a light-on time and a light-on timing of the LED 11; and power supply ON/OFF information for switching the power supply of each unit between an active mode in which each unit acquires intra-subject information and a standby mode in which each unit stops acquiring the intra-subject information, by controlling the system control circuit 23.

As described above, the power receiving antenna 17 of the capsule endoscope 2 receives the radio-transmitted power supply signal. The frame-number change information signal as the control information signal separated from the power supply signal by the separating circuit 18 is input to a control-information detecting circuit 24. A frequency band of the control information signal is made different from a frequency band of the power supply signal. A frequency band of the control information signal is changed depending on the content of the control.

Accordingly, the receiving apparatus 3 can radio transmit a multiplexed signal. In the capsule endoscope 2, the separating circuit 18 can separate the input signal, based on the frequency band. The control-information detecting circuit 24 can detect the content of the control information. The control-information detecting circuit 24 which receives the control information signal detects that the signal is a frame-number change information signal, and controls the CCD driving circuit 14 to drive the CCD 13 at the second imaging rate, according to the control content (a change from the first imaging rate to the second imaging rate).

So long as the control-information input unit 42 does not input new information, the superimposing circuit 43 continues superimposing the same control information on the power supply signal, for at least a predetermined period of time. Therefore, even when there is a period when the capsule endoscope 2 cannot temporarily receive power, the capsule endoscope 2 can receive the control information. Because, after the insertion of the capsule endoscope 2 into the subject 1, the function of the capsule endoscope 2 can be radio controlled from the outside, it is possible to prevent acquiring too much intra-subject information of an unnecessary part, and to prevent careless consumption of power.

The control circuit 50 detects the received field strength during the image receiving period, by relating the strength to the selected one of the receiving antennas A1 to An. The control circuit 50 outputs an instruction signal to the power-supply control circuit 40 so as to switch to the power supply antenna disposed near the receiving antenna that receives the largest received field strength, among the power supply antennas B1 to Bm that transmit the power supply signal. The power-supply control circuit 40 switch controls the switching circuit 45, based on the instruction signal, and switches the power supply antenna that transmits the power supply signal.

As explained above, in the present embodiment, the remaining-power-amount information of the capacitor of the capsule endoscope is superimposed on a transmission signal, and the superimposed signal is transmitted to the receiving apparatus at the outside of the subject. The receiving apparatus detects the remaining-power-amount information, and displays the information on the display unit. Therefore, the user can promptly recognize the remaining power amount within the capsule endoscope.

### Second Embodiment

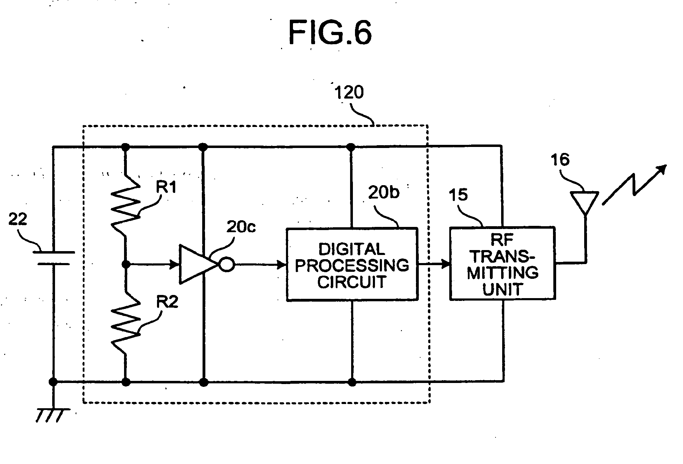

FIG. 6 is a block diagram of a configuration of a remaining-power-amount recognizing circuit 120 according to a second embodiment in accordance with the claimed invention, corresponding to the remaining-power-amount recognizing circuit 20 of the first embodiment shown in FIG. 2, and its periphery. The remaining-power-amount recognizing circuit 120 is different from the remaining-power-amount recognizing circuit 20 according to the first embodiment in that an inverter 20c is provided in place of the A/D converter, and that a voltage of the capacitor 22 is resistance-divided to become close to a threshold value of the inverter 20c, and the resistance-divided voltage is input to the inverter 20c. When the divided voltage becomes lower than the threshold value of the inverter 20c, a high-level flag signal is output from the inverter 20c to the digital processing circuit 20b. The digital processing circuit 20b superimposes a signal that shows that the flag signal attains a high level, on the transmission signal, and outputs the superimposed signal to the RF transmitting unit 15.

On receiving a signal indicating that the flag signal attains a high level, the receiving apparatus 3 radio transmits a power supply signal at a constant amplification level to the capsule endoscope 2, thereby making it possible to externally charge the capsule endoscope 2.

As explained above, according to the present embodiment, a state of the remaining power amount in the capacitor of the capsule endoscope is determined according to a binary value of a high level and a low level, based on the threshold value of the inverter. Therefore, an A/D converter having a large circuit scale does not need to be used. Consequently, the circuit scale of the receiving apparatus can be made small, and weight can be decreased. As a result, the load of the subject can be decreased when the subject carries the receiving apparatus.

### INDUSTRIAL APPLICABILITY

As explained above, the intra-subject information acquiring system according to the present invention is useful for a medical observation apparatus that is inserted into the human body for an observation of an examined part of a subject, and is particularly suitable to promptly recognize a remaining power amount of a body-insertable apparatus.

## Claims

1. An intra-subject information acquiring system comprising:
a body-insertable apparatus (2) that includes
a function executing unit (11, 13, 23) that executes a predetermined function of acquiring intra-subject information inside a subject (1),
a power supply unit (23) that supplies power to the function executing unit (11, 13, 23),
a power storage unit (22) that secures the power supply by radio power supply from outside, and
a transmitting unit (15, 16) that transmits a transmission signal that includes the intra-subject information acquired by the function executing unit (11, 13, 23) to outside the subject (1); and
an external device (32) that includes
a receiving unit (46) that receives the transmission signal transmitted from the transmitting unit (15, 16), **characterized in that**
the body-insertable apparatus (2) further includes
a recognizing unit (20) that recognizes a remaining power amount of the power supply unit (23), the recognizing unit (20) includes an inverter (20c), and determines the remaining power amount according to a binary value provided by the inverter (20c), and
a superimposing unit (20) that superimposes remaining-power-amount information recognized by the recognizing unit (20) on the transmission signal; and
the external device (32) further includes
a detector (48) that detects the remaining-power-amount information from the transmission signal received by the receiving unit (46), and
a displaying unit (51) that displays the remaining-power-amount information detected by the detector (48).

2. The intra-subject information acquiring system according to claim 1, wherein
the recognizing unit (20) further includes a resisitor that resistance-divides a voltage of the power storage unit (22) to make the resistance-divided voltage close to a threshold value of the inverter (20c), and
the inverter (20c) receives an input of the resistance-divided voltage and outputs a high-level flag signal to the transmitting unit (15, 16) when the resistance-divided voltage is lower than the threshold.

## Patentansprüche

1. Inner-Proband-Informationserfassungssystem aufweisend:
eine in einen Körper einfügbare Vorrichtung (2) enthaltend
eine Funktionsausführungseinheit (11, 13, 23), die eine vorbestimmte Funktion eines Erfassens von Inner-Proband-Information in einem Probanden (1) ausführt,
eine Energiezuführungseinheit (23), die der Funktionsausführungseinheit (11, 13, 23) Energie zuführt,
eine Energiespeichereinheit (22), welche die Energieversorgung durch Funkleistungszufuhr von außerhalb sichert und
eine Sendeeinheit (15, 16), die nach außerhalb des Probanden (1) ein Sendesignal sendet, welches die durch die Funktionsausführungseinheit (11, 13, 23) erfasste Intra-Proband-Information enthält; und
eine externe Vorrichtung (32) enthaltend
eine Empfangseinheit (46), die das von der Sendeeinheit (15, 16) gesendete Sendesignal empfängt, **dadurch gekennzeichnet, dass**
die in den Körper einfügbare Vorrichtung (2) ferner enthält
eine Erkennungseinheit (20), die eine Menge verbleibender Energie der Energiezuführungseinheit (23) erkennt, wobei die Erkennungseinheit (20) einen Inverter (20c) enthält und die Menge verbleibender Energie gemäß einem durch den Inverter (20c) bereitgestellten Binärwert bestimmt und
eine Überlagerungseinheit (20), welche die durch die Erkennungseinheit (20) erkannte Information der Menge verbleibender Energie dem Sendesignal überlagert; wobei
die externe Vorrichtung (32) ferner enthält
einen Detektor (48), der die Information der Menge verbleibender Energie von dem durch die Empfangseinheit (46) empfangenen Sendesignal erfasst und
eine Anzeigeeinheit (51), welche die durch den Detektor (48) erfasste Information der Menge verbleibender Energie anzeigt.

2. Inner-Proband-Informationserfassungssystem nach Anspruch 1, wobei
die Erkennungseinheit (20) ferner einen Widerstand enthält, der eine Spannung der Energiespeichereinheit (22) widerstandsteilt, so dass sich die widerstandsgeteilte Spannung einem Schwellwert des Inverters (20c) nähert, wobei
an den Inverter (20c) als Eingang die widerstandgeteilte Spannung angelegt wird und der Inverter (20c) an die Sendeeinheit (15, 16) ein Hoch-Flag-Signal ausgibt wenn die widerstandsgeteilte Spannung kleiner als der Schwellwert ist.

## Revendications

1. Système d'acquisition d'informations intra-sujet comprenant :
un appareil (2) insérable dans un corps qui inclut
une unité d'exécution de fonction (11, 13, 23) qui exécute une fonction prédéterminée d'acquisition d'informations intra-sujet à l'intérieur d'un sujet (1),
une unité d'alimentation en énergie (23) qui alimente en énergie l'unité d'exécution de fonction (11, 13, 23),
une unité de stockage d'énergie (22) qui sécurise l'alimentation en énergie par alimentation en énergie radio de l'extérieur, et
une unité de transmission (15, 16) qui transmet un signal de transmission qui inclut les informations intra-sujet acquises par l'unité d'exécution de fonction (11, 13, 23) vers l'extérieur du sujet (1) ; et
un dispositif externe (32) qui inclut
une unité de réception (46) qui reçoit le signal de transmission transmis par l'unité de transmission (15, 16), **caractérisé en ce que** l'appareil (2) insérable dans un corps inclut en outre
une unité de reconnaissance (20) qui reconnaît une quantité d'énergie restante de l'unité d'alimentation en énergie (23), l'unité de reconnaissance (20) inclut un inverseur (20c), et détermine la quantité d'énergie restante en fonction d'une valeur binaire fournie par l'inverseur (20c), et
une unité de superposition (20) qui superpose l'information de quantité d'énergie restante reconnue par l'unité de reconnaissance (20) sur le signal de transmission ; et
le dispositif externe (32) inclut en outre
un détecteur (48) qui détecte l'information de quantité d'énergie restante à partir du signal de transmission reçu par l'unité de réception (46), et
une unité d'affichage (51) qui affiche l'information de quantité d'énergie restante détectée par le détecteur (48).

2. Système d'acquisition d'informations intra-sujet selon la revendication 1, dans lequel
l'unité de reconnaissance (20) inclut en outre une résistance qui divise en résistance une tension de l'unité de stockage d'énergie (22) pour rendre la tension divisée en résistance proche d'une valeur de seuil de l'inverseur (20c), et
l'inverseur (20c) reçoit une entrée de la tension divisée en résistance et délivre en sortie un signal fanion de niveau haut à l'unité de transmission (15, 16) lorsque la tension divisée en résistance est inférieure au seuil.
